# EUROPEAN PATENT APPLICATION

(11) **EP 4 643 862 A1**
(43) Date of publication of application: **05.11.2025**
(21) Application number: 23910751.9
(22) Date of filing: 27.12.2023
(51) Int. Cl.: A61K 31/519, A61P 35/00

(54) **PHARMACEUTICAL COMPOSITION AND USE THEREOF**

(30) Priority: 28.12.2022 CN 202211702912; 21.04.2023 CN 202310440700
(71) Applicant: Guangzhou Joyo Pharmatech Co., Ltd., Guangzhou, Guangdong 510663 (CN); Shanghai Jia Tan Pharmatech Co. Ltd., Shanghai 201203 (CN)
(72) Inventor: LI, Yongguo, Guangzhou, Guangdong 510663 (CN); WEI, Wei, Shanghai 201203 (CN); LV, Jing, Shanghai 201203 (CN); YE, Wei, Shanghai 201203 (CN)
(74) Representative: Murgitroyd & Company
(86) International application number: PCT/CN2023/142441
(87) International publication number: WO 2024/140833

(57) **Abstract**

Disclosed are a pharmaceutical composition and use thereof. The pharmaceutical composition comprises compound I or a pharmaceutically acceptable salt thereof; and toripalimab. Compared with a single drug, the pharmaceutical composition of the present invention shows a better anti -tumor effect.

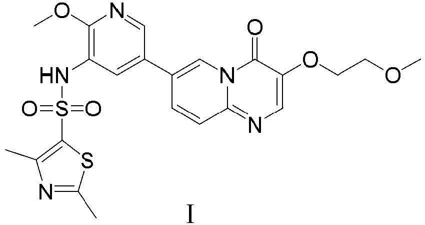

## Description

The present application claims the priority rights of Chinese patent application 2022117029124, filed on December 28, 2022, and Chinese patent application 2023104407001, filed on April 21, 2023. The contents of the above Chinese patent applications are incorporated herein by reference in their entirety.

### TECHNICAL FIELD

The present disclosure belongs to the field of medicine and specifically relates to a pharmaceutical combination and use thereof.

### BACKGROUND

Currently, tumors still remain as a major public health problem worldwide. With the understanding of tumor biology, the biological characteristics of tumors are gradually being analyzed, such as obtaining signals to maintain proliferation, escaping the regulation of growth inhibitory factors, continuously activating replication checkpoints, reprogramming cell metabolism, activating invasion and metastasis, *etc.* Targeting these biological characteristics of tumors, traditional chemotherapy, radiotherapy and targeted drug therapy have achieved good clinical therapeutic effects in the treatment of various types of tumors.

Immune checkpoint programmed death 1 (PD-1) is a transmembrane protein expressed on the surface of T cells, while programmed death-ligand 1 (PD-L1) is the ligand molecule of PD-1 expressed on the surface of tumor cells, APCs and T cells. When PD-L1 binds to PD-1, T cell response would be reduced by inhibiting the downstream signaling pathways of TCR activation. Antibodies targeting these immune checkpoints differ from traditional tumor treatments in that they play a role in killing tumors by enhancing the body's own immune system. Currently, antibodies targeting PD-1 and PD-L1, such as Nivolumab, Atezolizumab, Pembrolizumab, Durvalumab and Toripalimab, have achieved good results in the immunotherapy of various malignant tumors.

Toripalimab, developed by Junshi Biosciences, is the first monoclonal antibody drug approved for marketing in China targeting PD-1, and has been approved for various indications: melanoma, nasopharyngeal carcinoma, urothelial carcinoma and esophageal squamous carcinoma. Although Toripalimab has achieved good results in the treatment of certain malignant tumors, its efficacy still needs to be further improved.

### CONTENT OF THE PRESENT INVENTION

The present disclosure aims to provide a pharmaceutical combination of Compound I and Toripalimab and use thereof. Compared with a single drug, the pharmaceutical combination of the present disclosure shows better anti-tumor effect.

In a first aspect, the present disclosure provides a pharmaceutical combination comprising:
substance X, wherein the substance X is Compound I or a pharmaceutically acceptable salt thereof; and,
substance Y, wherein the substance Y is Toripalimab;

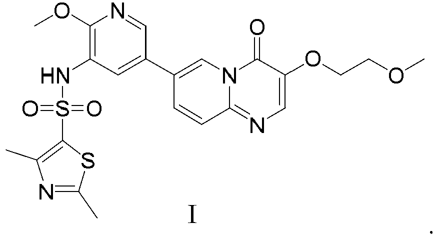

In the pharmaceutical combination, the substance X and the substance Y may be administered simultaneously or separately.

The "administered simultaneously" is, such as, that "a separate pharmaceutical composition comprising substance X" and "a separate pharmaceutical composition comprising substance Y" are administered simultaneously.

The "administered separately" is, such as, that "a separate pharmaceutical composition comprising substance X" and "a separate pharmaceutical composition comprising substance Y" are administered separately at different times, for example, one of "a separate pharmaceutical composition comprising substance X" and "a separate pharmaceutical compositions comprising substance Y" is administered firstly and the other is administered later. The "administered separately" may be temporally close or temporally distant.

Whether administered simultaneously or separately, the administration regimens of component X and component Y (including route of administration, frequency of administration, dosage of administration, interval of administration , *etc*.) may be the same or different, and may be adjusted as needed by those skilled in the art to provide the optimal therapeutic effect.

In some embodiments, the substance X is administered orally.

In some embodiments, the substance Y is administered by injection (*e.g.*, intravenous injection, subcutaneous injection, intraperitoneal injection, or intramuscular injection).

In some embodiments, the substance X is administered orally; and, the substance Y is administered by injection (*e.g.*, intravenous injection, subcutaneous injection, intraperitoneal injection, or intramuscular injection).

In another aspect, the present disclosure also provides a pharmaceutical combination comprising:
a first pharmaceutical composition comprising substance X and a pharmaceutical excipient, wherein the substance X is Compound I or a pharmaceutically acceptable salt thereof; and,
a second pharmaceutical composition comprising substance Y and a pharmaceutical excipient, wherein the substance Y is Toripalimab.

The first pharmaceutical composition is a separate pharmaceutical composition; the second pharmaceutical composition is a separate pharmaceutical composition.

In some embodiments, the first pharmaceutical composition is presented in an oral dosage form.

In some embodiments, the second pharmaceutical composition is presented in an injectable (*e.g.*, intravenous injection, subcutaneous injection or intramuscular injection) dosage form.

In some embodiments, the first pharmaceutical composition is presented in an oral dosage form; and the second pharmaceutical composition is presented in an injectable dosage form.

In another aspect, the present disclosure provides a medical kit comprising:
a first container comprising the first pharmaceutical composition as described above; and,
a second container comprising the second pharmaceutical composition as described above.

In another aspect, the present disclosure provides a use of the pharmaceutical combination as described herein in the manufacture of a medicament for treating cancer;
the cancer is preferably colon cancer, lung cancer, cervical cancer, head and neck cancer, nasopharyngeal carcinoma or urothelial carcinoma, and more preferably colon cancer.

In another aspect, the present disclosure provides a use of substance X in the manufacture of a medicament for treating cancer, wherein the substance X is Compound I or a pharmaceutically acceptable salt thereof, wherein the substance X is used in combination with substance Y, wherein the substance Y is Toripalimab, the cancer is preferably colon cancer, lung cancer, cervical cancer, head and neck cancer, nasopharyngeal carcinoma or urothelial carcinoma, and more preferably colon cancer;
when the cancer is lung cancer, the lung cancer is preferably non-small cell lung cancer;
when the cancer is head and neck cancer, the head and neck cancer is preferably head and neck squamous cell carcinoma;
when the cancer is cervical cancer, the cervical cancer is preferably cervical adenocarcinoma or cervical squamous cell carcinoma.

In another aspect, the present disclosure provides a use of substance Y in the manufacture of a medicament for treating cancer, wherein the substance Y is Toripalimab, wherein the substance Y is used in combination with substance X, the substance X is Compound I or a pharmaceutically acceptable salt thereof, the cancer is preferably colon cancer, lung cancer, cervical cancer, head and neck cancer, nasopharyngeal carcinoma or urothelial carcinoma, and more preferably colon cancer;
when the cancer is lung cancer, the lung cancer is preferably non-small cell lung cancer;
when the cancer is head and neck cancer, the head and neck cancer is preferably head and neck squamous cell carcinoma;
when the cancer is cervical cancer, the cervical cancer is preferably cervical adenocarcinoma or cervical squamous cell carcinoma.

In another aspect, the present disclosure provides a method for treating cancer, which comprises administering a therapeutically effective amount of the pharmaceutical combination as described herein to a subject (e.g., human or mouse) in need thereof, the cancer is preferably colon cancer, lung cancer, cervical cancer, head and neck cancer, nasopharyngeal carcinoma or urothelial carcinoma, and more preferably colon cancer;
when the cancer is lung cancer, the lung cancer is preferably non-small cell lung cancer;
when the cancer is head and neck cancer, the head and neck cancer is preferably head and neck squamous cell carcinoma;
when the cancer is cervical cancer, the cervical cancer is preferably cervical adenocarcinoma or cervical squamous cell carcinoma.

### Administration of substance X and substance Y

In some embodiments, the administration regimens of substance X and substance Y (including route of administration, dosage of administration, interval of administration, etc.) may be the same or different, and may be adjusted as needed by those skilled in the art to provide the optimal therapeutic effect.

In some embodiments, the substance X and the substance Y may be administered simultaneously or separately.

In some embodiments, the substance X is administered orally.

In some embodiments, the substance Y is administered by injection (e.g., intravenous injection, subcutaneous injection, intraperitoneal injection, or intramuscular injection).

In some embodiments, the substance X is administered orally; and, the substance Y is administered by injection (*e.g.*, intravenous injection, subcutaneous injection, intraperitoneal injection, or intramuscular injection).

In some embodiments, the frequency of administration of the substance X can be QD (once a day), QOD (every other day) or BID (twice a day), preferably QD.

In some embodiments, the amount of the substance X to be administered each time can be determined according to the body weight of the subject, and non-limiting examples can range from 0.1 mg/kg to 1 mg/kg (referring to a single dose), such as 0.2 mg/kg; in some embodiments, the dose of Compound I is 0.2 mg/kg.

In some embodiments, the administration dose of the substance X is 0.1 mg -2.0 mg/dose, for example: 0.1 mg/dose, 0.2 mg/dose, 0.3 mg/dose, 0.4 mg/dose, 0.5 mg/dose, 0.6 mg/dose, 0.7 mg/dose, 0.8 mg/dose, 0.9 mg/dose, 1.0 mg/dose, 1.1 mg/dose, 1.2 mg/dose, 1.3 mg/dose, 1.4 mg/dose, 1.5 mg/dose, 1.6 mg/dose, 1.7 mg/dose, 1.8 mg/dose, 1.9 mg/dose or 2.0 mg/dose, preferably 1.1 mg/dose.

In some embodiments, the substance X is orally administered at the above dosage and frequency; for example, the substance X is orally administered at 0.2 mg/kg, QD. In some embodiments, the substance X is orally administered at 1.1 mg/dose, QD.

The substance Y can be administered according to the body weight of the subject, and non-limiting examples can range from 0.1-10 mg/kg (referring to a single dose), preferably 1-5 mg/kg, such as 1 mg/kg, 2 mg/kg, 3 mg/kg, 4 mg/kg or 5 mg/kg; in some embodiments, the dose of Toripalimab is 1 mg/kg.

In some embodiments, administration dose of the substance Y is 6 to 600 mg/dose, for example, 40 mg/dose, 80 mg/dose, 120 mg/dose, 160 mg/dose, 200 mg/dose, 240 mg/dose, 280 mg/dose, 320 mg/dose, 360 mg/dose, 400 mg/dose, 440 mg/dose, 480 mg/dose, 520 mg/dose, 560 mg/dose or 600 mg/dose, preferably 240 mg/dose.

The frequency of administration of the substance Y can be QW (once a week), BIW (twice a week), Q2W (once every two weeks) or Q3W (once every three weeks).

In some embodiments, the substance Y is administered at a frequency of BIW.

In some embodiments, the substance Y is administered by injection at the above dosage and frequency; for example, the substance Y is administered by injection at 1 mg/kg, BIW. In some embodiments, the substance Y is administered by injection, at 240 mg/dose, Q3W.

When substance X and substance Y are administered separately, they can be administered continuously according to their respective dosing periods. The dosing periods of substance X and substance Y may begin at the same time or at different times. For example, substance X and substance Y may be administered continuously according to their respective dosing periods starting on the same day; or, substance X may be started to be administered on the second, third or more days after the start of administration of substance Y, and then both are administered continuously according to their respective dosing periods.

As used herein, the term "treatment" refers to therapeutic therapy. In the context of specific diseases, "treatment" refers to: (1) alleviating one or more biological manifestations of a disease or condition; (2) interfering with (a) one or more points in the biological cascade leading to or causing the condition, or (b) one or more biological manifestations of the condition; (3) ameliorating one or more symptoms, effects, or side effects associated with the condition, or one or more symptoms, effects, or side effects associated with the condition or its treatment, or (4) delaying the progression of one or more biological manifestations of the disease or condition.

As used herein, the term "therapeutically effective amount" refers to the quantity of the compound that, when administered to a subject, is sufficient to effectively treat the disease or condition described herein. The amount of a compound constituting a "therapeutically effective amount" will vary based on the compound, the disease and its severity, as well as the age of the subject to be treated, and can be adjusted as needed by those skilled in the art.

The term "subject" as used herein refers to any animal that is to be or has been administered the compound or composition in accordance with embodiments of the present disclosure, with mammals being preferred, and humans beings most preferred. As used herein, the term "mammal" includes any mammal. Examples of mammals include, but are not limited to, cattle, horses, sheep, pigs, cats, dogs, mice, rats, rabbits, guinea pigs, monkeys, humans, *etc*., with humans being most preferred.

As used herein, the term "pharmaceutically acceptable salt" refers to a salt prepared by the compound with a relatively nontoxic, pharmaceutically acceptable acid or base. When the compound contains a relatively acidic functional group, a base addition salt can be obtained by bringing the neutral form of such compound into contact with a sufficient amount of a pharmaceutically acceptable base in a pure solution or a suitable inert solvent.

As used herein, the term "container" refers to any container and closure suitable for storing, transporting, dispensing and/or handling pharmaceutical products.

The term "pharmaceutical excipient" used herein refers to excipient and additive used in the manufacture of pharmaceutical products and in the formulation of prescriptions, and are all substances, other than the active ingredient, that are included in a pharmaceutical preparation. Please refer to the Pharmacopoeia of the People's Republic of China (2020 Edition), Part IV, or Handbook of Pharmaceutical Excipients (Raymond C Rowe, 2009 Sixth Edition).

Without departing from the common understanding in this field, the aforementioned preferred conditions can be freely combined to obtain the preferred examples of the present disclosure.

The reagents and raw materials used in the present disclosure are all commercially available.

The positive progressive effects of the present disclosure are that: the present disclosure provides a pharmaceutical combination of Compound I and Toripalimab, which shows a better anti-tumor effect compared to a single drug.

### BRIEF DESCRIPTION OF THE DRAWINGS

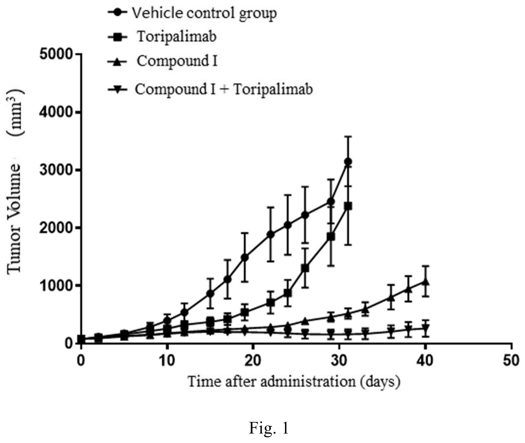

Fig. 1 is the growth curve of tumor volume in mice.

### DETAILED DESCRIPTION OF THE EMBODIMENT

The present disclosure will be further illustrated by way of examples below, but the present disclosure should not be limited to the scope of these examples. The experimental methods for which the specific conditions are not specified in the following examples are selected according to the conventional methods and conditions, or according to the commodity instructions.

### Example 1: Anti-tumor effect of Compound I in combination with Toripalimab in subcutaneous transplantation tumor of mouse colon cancer cells CT26

### 1.1 Experimental materials:

BALB/c-hPD1 mice, female, 6.9 weeks (weekly age when inoculation), were provided by Jiangsu GemPharmatech Co., Ltd.

Toripalimab was provided by Shanghai Junshi Biosciences Co., Ltd.

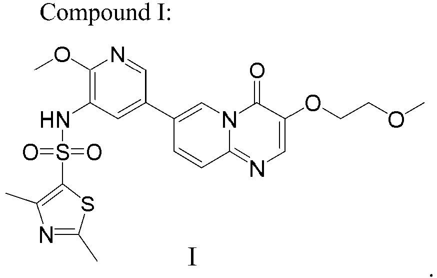

### 1.2 Experimental model

BALB/c-hPD1 mice were subcutaneously inoculated with CT26 cells, and CT26 cells were provided by Jiangsu GemPharmatech Co., Ltd.

### 1.3 Cell culture

The mycoplasma test result of CT26 cells was negative, and the recovery generation was Pn+8. Mouse colon cancer CT26 cells were cultured *in vitro* in monolayer, in RPMI1640 medium added with 10% (v/v) fetal bovine serum, 100 U/mL penicillin, and 100 µg/mL streptomycin, at 37°C, 5% CO₂, 95% relative humidity, digested with trypsin for passage according to the cell density, and when the cells were in the logarithmic growth phase, the cells were digested for inoculation.

### 1.4 Tumor inoculation

CT26 cells in the logarithmic growth phase (inoculation passage is Pn+11) were collected, the culture medium was removed and the cells were washed twice with DPBS before inoculation (the cell survival rates before and after tumor bearing were 94.31% and 91.43%, respectively). The inoculation amount was 5×10⁵ cells/100 µL/mouse.

### 1.5 Grouping

When the tumor volume reached 80.15 mm³, the mice were randomly divided into groups as shown in Table 1 below according to the tumor volume. The day of grouping was defined as D0.

**Table 1 Administration regimen**

| Group | Number of animals (individuals) | Treatment Group | Dose (mg/kg) | Route of administration | Frequency period |
|---|---|---|---|---|---|
| **1** | 5 | Vehicle control group | - | Intraperitoneal | Twice a week |
| **2** | 5 | Toripalimab^{a} | 1 | Intraperitoneal | Twice a week |
| **3** | 5 | Compound I^{b} | 0.2 | Oral | Once a day |
| **4** | 5 | Compound I^{b} | 0.2 | Oral | Once a day |
| | | Toripalimab^{a} | 1 | Intraperitoneal | Twice a week |

| | | | | | |
|---|---|---|---|---|---|
| Note: 1) The vehicle control group was normal saline. 2) a, the vehicle used was normal saline. 3) b, the vehicle used is 1% DMSO + 99% (1% methylcellulose). | | | | | |

### 1.6 Drug administration

Drug administration began at D0, with a volume of 10 µL/g × mouse body weight (g). The administration regimen is shown in Table 1. For the combination administration group, each drug was administered separately with unchanged drug concentration and doubled drug volume. The order of administration remained consistent for each administration.

### 1.7 Observation

In the early stage, the tumors of the mice were measured twice a week and the body weight was weighed twice a week. In the later stage, the tumors grew faster, so the observation frequency was adjusted to three times a week. After 40 days of observation, the tumor growth curve (as shown in Fig. 1) was obtained and analyzed.

### 1.8. Experimental results

The tumor growth obtained after 40 days is shown in Table 2 below.

**Table 2**

| Group | | Vehicle control group | Toripalimab | Compound I | Compound I + Toripalimab |
|---|---|---|---|---|---|
| | D0 | 80.21 ± 3.05 | 80.14 ± 3.36 | 80.11 ± 1.52 | 80.14 ± 2.90 |
| | D2 | 115.86 ± 18.70 | 104.91 ± 10.47 | 94.96 ± 0.67 | 101.16 ± 4.43 |
| | D5 | 177.26 ± 41.42 | 163.18 ± 31.86 | 124.52 ± 5.59 | 136.33 ± 12.82 |
| | D8 | 289.33 ± 71.75 | 217.55 ± 45.42 | 152.48 ± 8.28 | 156.09 ± 19.23 |
| | D10 | 397.95 ± 109.49 | 259.84 ± 55.46 | 180.37 ± 11.60 | 171.86 ± 23.82 |
| | D12 | 542.56 ± 157.16 | 328.01 ± 67.16 | 203.71 ± 10.99 | 191.71 ± 33.88 |
| | D15 | 866.61 ± 255.68 | 376.10 ± 78.06 | 230.42 ± 12.46 | 206.88 ± 32.85 |
| | D17 | 1113.34 ± 333.01 | 424.59 ± 101.56 | 248.64 ± 14.78 | 202.84 ± 41.11 |
| | D19 | 1491.88 ± 421.43 | 541.36 ± 141.45 | 263.11 ± 15.61 | 199.15 ± 48.56 |
| Tumor volume (mm³) | D22 | 1888.64 ± 466.07 | 710.20 ± 191.06 | 286.35 ± 21.17 | 192.79 ± 54.66 |
| | D24 | 2052.23 ± 517.82 | 874.27 ± 229.05 | 316.22 ± 28.82 | 177.49 ± 66.85 |
| | D26 | 2226.02 ± 488.70 | 1307.59 ± 340.32 | 392.45 ± 59.16 | 164.72 ± 76.31 |
| | D29 | 2456.61 ± 379.25 | 1854.39 ± 507.92 | 462.29 ± 80.77 | 159.59 ± 83.47 |
| | D31 | 3147.52 ± 430.65 | 2382.11 ± 676.42 | 518.88 ± 91.26 | 163.44 ± 87.65 |
| | D33 | - | - | 598.33 ± 116.39 | 171.89 ± 92.87 |
| | D36 | - | - | 803.27 ± 211.15 | 208.73 ± 109.50 |
| | D38 | - | - | 949.17 ± 219.86 | 243.82 ± 130.79 |
| | D40 | - | - | 1079.32 ± 262.97 | 263.87 ± 143.99 |

| | | | | | |
|---|---|---|---|---|---|
| Note: "-" means no test data | | | | | |

According to the statistical analysis of D22 tumor volume data, the tumor volumes of the group of Toripalimab (TGI_{TV}=62.16%, tumor growth inhibition rate=1-tumor weight of experimental group/tumor weight of control group), group of Compound I (TGI_{TV}=84.57%), combination group of Toripalimab and Compound I (TGI_{TV} = 89.55%) was significantly different from that of vehicle control group (P < 0.05 or P < 0.01). According to the statistical analysis of D31 tumor volume data, there was a significant difference in tumor volume between the combination group of Toripalimab and Compound I (TGI_{TV}=94.63%), Toripalimab single drug group (TGI_{TV}=24.42%), and Compound I single drug group (TGI_{TV}=83.37%) (P<0.05). According to the statistical analysis of tumor volume data on day 40, there was a significant difference in tumor volume between the combination group of Toripalimab and Compound I, and Compound I single drug group (P<0.05).

In summary, the results of this experiment showed that in subcutaneous transplantation tumor of mouse colon cancer cells CT26 model, compared with the normal saline in the control group, Toripalimab single drug, Compound I single drug, and the combination of Compound I and Toripalimab all showed tumor inhibitory effects; compared with Toripalimab single drug and Compound I single drug, the combination administration of Compound I and Toripalimab showed better tumor inhibitory effects.

### Example 2: Efficacy data of Compound I combined with Toripalimab in cervical adenocarcinoma in clinical trials.

Case information: Subject 1, female, 69 years old, pathology report on October 28, 2022 showed cervical adenocarcinoma; informed consent was given on November 11, 2022 to participate in the "Phase Ib/II clinical trial to evaluate the safety, tolerability and preliminary efficacy of Compound I combined with Toripalimab in advanced solid tumors": treatment started on November 18, 2022, Compound I tablets, dose of which is 0.7 mg, single dose administration, once a day, 0.7 mg dose containing one tablet of Compound I 0.5 mg and two tablets of Compound I 0.1 mg; the dose of Toripalimab is 240 mg, which is administered intravenously every three weeks. The baseline target lesion was a fused lymph node adjacent to the left external iliac vessels, with the longest diameter of 111 mm. After the combination of Compound I and Toripalimab, the therapeutic effect was significant with sustained tumor shrinkage. The maximum tumor shrinkage ratio was 17.1% and lasted for 8 periods (21 days per period).

### Example 3: Efficacy data of Compound I combined with Toripalimab in head and neck squamous cell carcinoma in clinical trials.

Case information: Subject 2, male, 59 years old, underwent "subtotal laryngectomy (supracircular)" on October 28, 2020; postoperative pathology report showed laryngeal cancer; postoperative radiotherapy was performed from November 30, 2020 to January 21, 2021, and MRI examination showed recurrence on November 15, 2022; informed consent was given on November 17, 2022 to participate in the "Phase Ib/II clinical trial to evaluate the safety, tolerability and preliminary efficacy of Compound I combined with Toripalimab in advanced solid tumors": treatment started on November 25, 2022, Compound I tablets, dose of which is 0.7 mg, single dose administration, once a day, 0.7 mg dose containing one tablet of Compound I 0.5 mg and two tablets of Compound I 0.1 mg; the dose of Toripalimab is 240 mg, which is administered intravenously every three weeks. The baseline target lesion was the larynx, with the longest diameter of 34 mm. After the combination of Compound I and Toripalimab, the therapeutic effect was significant, the tumor was reduced to less than 5mm, showing PR and 85.3% tumor shrinkage, and the subject's PR was lasted for more than 10 periods (21 days per period).

### Example 4: Efficacy data of Compound I combined with Toripalimab in non-small cell lung cancer in clinical trials.

Case information: Subject 3, male, 73 years old, pathology report showed non-small cell lung cancer; immunohistochemistry showed high expression of PD-L1, the results of tissue genetic testing showed EGFR/ALK double negative. Informed consent was given on July 21, 2023 to participate in the "Phase Ib/II clinical trial to evaluate the safety, tolerability and preliminary efficacy of Compound I combined with Toripalimab in advanced solid tumors": after receiving treatment, Compound I tablets, dose of which is 0.9 mg, single dose administration, once a day, 0.9 mg dose containing one tablet of Compound I 0.5 mg and four tablets of Compound I 0.1 mg; the dose of Toripalimab is 240 mg, which is administered intravenously every three weeks. After the combination of Compound I and Toripalimab, the therapeutic effect was significant, with tumor shrinkage of 48.2%, PR (partial remission, the sum of the maximum diameters of target lesions decreased by ≥30%, maintained for at least 4 weeks).

### Example 5: Efficacy data of Compound I combined with Toripalimab in urothelial carcinoma in clinical trials.

Case information: Subject 4, female, 71 years old, showed urothelial carcinoma of right ureter in pathology report in June 2023; and previously experienced first-line gemcitabine plus cisplatin resistance recurrence; informed consent was given on July 14, 2023 to participate in the "Phase Ib/II clinical trial to evaluate the safety, tolerability and preliminary efficacy of Compound I combined with Toripalimab in advanced solid tumors": treatment started on July 17, 2022, Compound I tablets, dose of which is 1.1 mg, single dose administration, once a day, 1.1 mg dose containing two tablets of Compound I 0.5 mg and one tablet of Compound I 0.1 mg; the dose of Toripalimab is 240 mg, which is administered intravenously every three weeks. The baseline target lesion was the left bladder wall and right arm, totaling 81.8 mm. After the combination of Compound I and Toripalimab, the therapeutic effect was significant, with sustained tumor shrinkage, maximum tumor shrinkage of 38.9 mm, tumor shrinkage of 52.4%, PR and lasting for at least 6 periods (21 days per period).

### Example 6: Efficacy data of Compound I combined with Toripalimab in nasopharyngeal carcinoma in clinical trials.

Case information: Subject 5, male, 54 years old, pathology report showed nasopharyngeal carcinoma. Recurrence after receiving radical radiotherapy in July-August 2022. Informed consent was given on February 13, 2023 to participate in the "Phase Ib/II clinical trial to evaluate the safety, tolerability and preliminary efficacy of Compound I combined with Toripalimab in advanced solid tumors": Treatment started on February 24, 2023, Compound I tablets, dose of which is 0.9 mg, single dose administration, once a day, 0.9 mg dose containing one tablet of Compound I 0.5 mg and four tablets of Compound I 0.1 mg; the dose of Toripalimab is 240 mg, which is administered intravenously every three weeks. The baseline target lesions were lymph nodes with a thickness of 17 layers (left axillary) of 26mm in portal phase and lymph nodes with a thickness of 19 layers of 18mm in portal phase, totaling 44mm. After the combination of Compound I and Toripalimab, the therapeutic effect was significant, with the tumor reaching PR (partial remission, reduction of the sum of the maximum diameters of the target lesions by ≥30%, lasted for at least 4 weeks) and lasting for more than 12 periods (21 days per period). During the treatment period, the tumor continued to shrink, with a maximum shrinkage ratio of 72.7%.

### Example 7: Efficacy data of Compound I combined with Toripalimab in cervical squamous cell carcinoma in clinical trials.

Case information: Subject 6, female, 59 years old, was initially diagnosed with cervical squamous cell carcinoma in March 2020 and received concurrent radiotherapy and chemotherapy; informed consent was given on March 31, 2023 to participate in the "Phase Ib/II clinical trial to evaluate the safety, tolerability and preliminary efficacy of Compound I combined with Toripalimab in advanced solid tumors": treatment started on April 07, 2023, Compound I tablets, dose of which is 1.1 mg, single dose administration, once a day, 1.1 mg dose containing two tablets of Compound I 0.5 mg and one tablet of Compound I 0.1 mg; the dose of Toripalimab is 240 mg, which is administered intravenously every three weeks. The baseline target lesion was the left cervical lymph node with the longest diameter of 15mm. After the combination of Compound I and Toripalimab, the therapeutic effect was significant, with sustained tumor shrinkage, maximum tumor shrinkage of 10 mm, and the tumor shrinkage ratio was 36.3% (PR) and lasted for 8 periods (21 days per period).

### Example 8: Efficacy data of Compound I combined with Toripalimab in cervical squamous cell carcinoma in clinical trials.

Case information: Subject 7, female, 75 years old, pathology report on March 31, 2023 showed cervical invasive squamous cell carcinoma; informed consent was given on April 04, 2023 to participate in the "Phase Ib/II clinical trial to evaluate the safety, tolerability and preliminary efficacy of Compound I combined with Toripalimab in advanced solid tumors": treatment started on April 07, 2023, Compound I tablets, dose of which is 1.1 mg, single dose administration, once a day, 1.1 mg dose containing two tablets of Compound I 0.5 mg and one tablet of Compound I 0.1 mg; the dose of Toripalimab is 240 mg, which is administered intravenously every three weeks. The baseline target lesion was the cervical region, with the longest diameter of 21mm. After the combination of Compound I and Toripalimab, the therapeutic effect was significant, with sustained tumor shrinkage, maximum tumor shrinkage of 5 mm (PR), tumor shrinkage of 76.2% and lasting for at least 10 periods (21 days per period).

### Example 9: Efficacy data of Compound I combined with Toripalimab in cervical squamous cell carcinoma in clinical trials.

Case information: Subject 8, female, 48 years old, pathology report on February 08, 2022 showed cervical keratinizing invasive squamous cell carcinoma; informed consent was given on June 02, 2023 to participate in the "Phase Ib/II clinical trial to evaluate the safety, tolerability and preliminary efficacy of Compound I combined with Toripalimab in advanced solid tumors": treatment started on June 09, 2023, Compound I tablets, dose of which is 1.1 mg, single dose administration, once a day, 1.1 mg dose containing two tablets of Compound I 0.5 mg and one tablet of Compound I 0.1 mg; the dose of Toripalimab is 240 mg, which is administered intravenously every three weeks. The baseline target lesion was the vaginal area with the longest diameter of 36 mm. After the combination of Compound I and Toripalimab, the therapeutic effect was significant with sustained tumor shrinkage, maximum tumor shrinkage ratio was 36.1% (23mm, PR) and lasted for at least 8 periods (21 days per period).

### Example 10: Efficacy data of Compound I combined with Toripalimab in head and neck squamous cell carcinoma in clinical trials.

Case information: Subject 9, male, 73 years old, was first diagnosed with right maxillary gingival squamous cell carcinoma in August 2021, and underwent enlarged resection of the right maxilla, local enlarged resection of the right palate tumor, and right cervical lymph node dissection in the same month. Previously, Subject 9 had received first-line treatment with carrelizumab and SI-B001 (EGFR/HER3 dual antibody) combined with paclitaxel, but developed drug resistance and relapse; informed consent was given on June 27, 2023 to participate in the "Phase Ib/II clinical trial to evaluate the safety, tolerability and preliminary efficacy of Compound I combined with Toripalimab in advanced solid tumors": Treatment started on July 02, 2023, Compound I tablets, dose of which is 1.1 mg, single dose administration, once a day, 1.1 mg dose containing two tablets of Compound I 0.5 mg and one tablet of Compound I 0.1 mg; the dose of Toripalimab is 240 mg, which is administered intravenously every three weeks. The baseline target lesion was the right buccal with a longest diameter of 30mm. After the combination of Compound I and Toripalimab, the therapeutic effect was significant with sustained tumor shrinkage, maximum tumor reduction ratio was 100% and lasted for at least 6 periods (21 days per period).

Although the foregoing describes specific embodiments of the present disclosure, it should be understood by those skilled in the art that these are provided only as examples. Various changes or modifications can be made to these embodiments without departing from the principles and essence of the present disclosure. Therefore, the scope of protection for the present disclosure is defined by the appended claims.

## Claims

1. A pharmaceutical combination comprising:
substance X, wherein the substance X is Compound I or a pharmaceutically acceptable salt thereof; and,
substance Y, wherein the substance Y is Toripalimab;

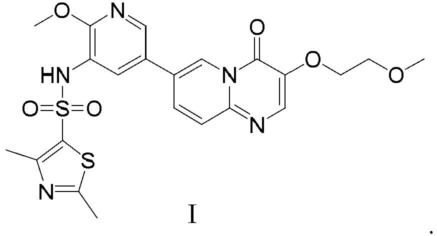

2. The pharmaceutical combination according to claim 1, wherein the pharmaceutical combination satisfies one or more of the following conditions:
(1) the substance X and the substance Y are administered simultaneously or separately;
(2) the substance X is administered orally;
(3) the substance Y is administered by injection.

3. A pharmaceutical combination comprising:
a first pharmaceutical composition comprising substance X and a pharmaceutical excipient, wherein the substance X is Compound I or a pharmaceutically acceptable salt thereof; and,
a second pharmaceutical composition comprising substance Y and a pharmaceutical excipient, wherein the substance Y is Toripalimab;

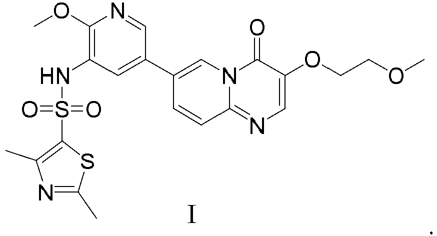

4. The pharmaceutical combination according to claim 3, wherein the pharmaceutical combination satisfies one or more of the following conditions:
(1) the first pharmaceutical composition is presented in an oral dosage form;
(2) the second pharmaceutical composition is presented in an injectable dosage form.

5. A medical kit comprising:
a first container comprising the first pharmaceutical composition according to claim 3 or 4; and,
a second container comprising the second pharmaceutical composition according to claim 3 or 4.

6. A use of the pharmaceutical combination according to any one of claims 1 to 4 in the manufacture of a medicament for treating cancer.

7. A use of substance X in the manufacture of a medicament for treating cancer, wherein the substance X is compound I or a pharmaceutically acceptable salt thereof, wherein, the substance X is used in combination with substance Y, and the substance Y is Toripalimab;

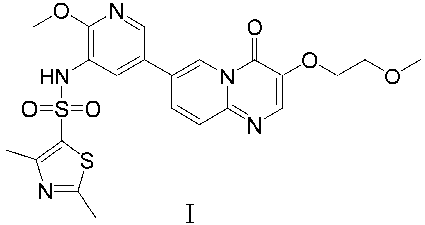

8. A use of substance Y in the manufacture of a medicament for treating cancer, wherein the substance Y is Toripalimab, wherein the substance Y is used in combination with substance X, and the substance X is Compound I or a pharmaceutically acceptable salt thereof;

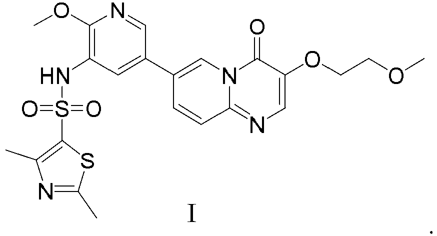

9. The use according to any one of claims 6 to 8, wherein the cancer is colon cancer, lung cancer, cervical cancer, head and neck cancer, nasopharyngeal carcinoma or urothelial carcinoma, preferably colon cancer;
when the cancer is lung cancer, the lung cancer is preferably non-small cell lung cancer;
when the cancer is head and neck cancer, the head and neck cancer is preferably head and neck squamous cell carcinoma;
when the cancer is cervical cancer, the cervical cancer is preferably cervical adenocarcinoma or cervical squamous cell carcinoma.

10. The use according to any one of claims 6 to 9, wherein the use satisfies one or more of the following conditions:
(1) the substance X and the substance Y can be administered simultaneously or separately;
(2) the substance X is administered orally;
(3) the substance Y is administered by injection;
(4) the substance X is administered according to the subject's body weight, with an administration dose of 0.1 mg/kg to 1 mg/kg, for example 0.2 mg/kg;
(5) administration dose of the substance X is 0.1 mg-2.0 mg/dose, for example, 0.1 mg/dose, 0.2 mg/dose, 0.3 mg/dose, 0.4 mg/dose, 0.5 mg/dose, 0.6 mg/dose, 0.7 mg/dose, 0.8 mg/dose, 0.9 mg/dose, 1.0 mg/dose, 1.1 mg/dose, 1.2 mg/dose, 1.3 mg/dose, 1.4 mg/dose, 1.5 mg/dose, 1.6 mg/dose, 1.7 mg/dose, 1.8 mg/dose, 1.9 mg/dose or 2.0 mg/dose, preferably 1.1 mg/dose;
(6) the frequency of administration of the substance X is QD, QOD or BID, for example QD;
(7) the substance Y is administered according to the subject's body weight, with administration dose of 0.1 mg/kg to 10 mg/kg, for example, 1 mg/kg to 5 mg/kg, for example, 1 mg/kg, 2 mg/kg, 3 mg/kg, 4 mg/kg or 5 mg/kg;
(8) administration dose of the substance Y is 6 -600 mg/dose, for example: 40 mg/dose, 80 mg/dose, 120 mg/dose, 160 mg/dose, 200 mg/dose, 240 mg/dose, 280 mg/dose, 320 mg/dose, 360 mg/dose, 400 mg/dose, 440 mg/dose, 480 mg/dose, 520 mg/dose, 560 mg/dose or 600 mg/dose, preferably 240 mg/dose;
(9) the frequency of administration of the substance Y is QW, BIW, Q2W or Q3W, for example, Q2W or Q3W.
